(19)

**Europäisches Patentamt**

**European Patent Office**

**Office européen des brevets**

(11) **EP 4 015 045 A1**

(12) **EUROPEAN PATENT APPLICATION**

(43) Date of publication:
**22.06.2022 Bulletin 2022/25**

(21) Application number: **20214423.4**

(22) Date of filing: **16.12.2020**

(51) International Patent Classification (IPC):
**A61Q 17/04** (2006.01)   **A61K 8/34** (2006.01)
**A61K 8/88** (2006.01)   **A61K 8/90** (2006.01)

(52) Cooperative Patent Classification (CPC):
**A61Q 17/04; A61K 8/34; A61K 8/88; A61K 8/90**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **IONIQ Skincare GmbH & Co. KG**
**88677 Markdorf (DE)**

(72) Inventors:
• **WILLE, Charlotte**
  **22927 Großhansdorf (DE)**
• **HARNISCH, Eva**
  **88214 Ravensburg (DE)**
• **LUECK, Annabella**
  **74906 Bad Rappenau (DE)**

• **GOEHRING, Alfred**
  **88682 Salem (DE)**
• **JELTSCH, Thomas**
  **88048 Friedrichshafen (DE)**
• **WEMHEUER, Niklas**
  **88690 Uhldingen-Muehlhofen (DE)**
• **FANOUS, Philipp**
  **88662 Ueberlingen (DE)**
• **LANGEN, Valentin**
  **88662 Ueberlingen (DE)**
• **GROß, Philipp**
  **71229 Leonberg (DE)**

(74) Representative: **Lederer & Keller Patentanwälte Partnerschaft mbB**
**Unsöldstraße 2**
**80538 München (DE)**

(54) **ELECTROSTATICALLY SPRAYABLE COSMETIC FORMULATION**

(57) The present invention relates to an electrostatically sprayable cosmetic formulation having a sufficient sun protection factor, water resistance and caring effect, and which provides a sufficient, i.e. nearly full, coverage of the skin surface, when the formulation is applied on the surface of the skin by electrostatic spraying.

**EP 4 015 045 A1**

**Description**

The present invention relates to an electrostatically sprayable cosmetic formulation, which can be easily applied on a subject, provides in particular an improved protection against harmful UV-A and UV-B radiation, a good water resistance and additionally a good caring effect.

It is well known that ultraviolet radiation of the sun having a wave length of about 280 to about 400 nm, i.e. UV-B (280 nm to 320 nm) and UV-A radiation (320 nm to 400 nm), is responsible for skin damages such as sunburn or cellular mutation, but also causes loss of skin elasticity, suppress immune functions, promote premature signs of aging and other undesirable health effects. On the other hand, for most of the people is it desirable to tan the skin for purpose of appearance. The tanning of the skin is caused by ultraviolet radiation of longer wave lengths of up to about 420 nm. In order to prevent or reduce the negative impacts of the sun radiation various sun screen products are on the market. In addition to this main function of the sun screen it is also desirable that the sun screen shows a good resistance against sweat, fresh or salt water in case the subject is exposed to water.

Nowadays, sun screens are commercial available having a high protection against UV-A and UV-B sun radiation and which show a good water resistance.

However, even though from a theoretical point of view the sun screen formulations known in the prior art show sufficient protection against sun radiation, in practice the protection against harmful sun radiations leaves much to be desired. One of the reasons for this is that the sun screen formulations are not applied on the surface of the skin in a correct manner.

Most of the sun screen formulations are lotions which can be applied on the surface of the skin by a limiting number of methods. The simple method is to apply the lotion by hand. This method includes many disadvantages. In particular, the method does not ensure that a sufficient amount of the sunscreen lotion is applied on the surface of the skin and in particular this application method does not provide a sufficient coverage of the skin. Moreover, in some cases unnecessary high amounts of the sun screen formulation are applied on skin surface which results into uncomfortable feeling.

Another application method, which should ensure that a sufficient amount of the sun screen formulation is applied on the surface of the skin, but in an adequate manner, is the use of pump sprays or pre-pressurized aerosol containers so as to have the formulation atomized and sprayed with the aid of propellant gas. However, conventional aerosol sprays frequently employ volatile compounds as propellants, which are environmentally unfriendly, possible hazardous to health and indeed are being legislated against in many countries. Moreover, it is hardly possible to generate a constant and homogenous flow of the sun screen formulation. Therefore, also with this application method a sufficient coverage of the skin surface, i.e. a nearly 100% coverage, is not possible.

In the last few years in the field of cosmetic products a further application method has become of interest. In this application method the cosmetic product is applied on the subject by electrostatic spraying. Such a method is for example described in WO 94/11119 or WO 2001/012139.

This application method can be carried out without the aid of environmentally unfriendly propellants and provides a constant and uniform flow of the product. Hence, it is possible to apply a sufficient amount of the cosmetic formulation, e.g. sun screen formulation, on for example the surface of the skin and thus a nearly full coverage of the skin may be possible.

In the prior art different electrostatic spraying devices are known, for example in WO 94/11119, US 2010/0116897 or DE 10 2017 108 610.2 such devices are described. In an electrostatic spaying device a voltage generator creates a high voltage which electrically charges the compounds of the cosmetic product. This results into a spray of the cosmetic product.

However, as pointed out in EP 19 197 645, which is incorporated herein by reference, the most problem of this application method is that the known cosmetic formulations such as sun screen formulations are not electrostatically sprayable, since they do not have appropriate electrical characteristics, e.g. resistivity, permittivity etc., and/or have other properties such like a high surface tension, viscosity etc. which do not permit electrostatic spraying. For example, like many other cosmetic lotions and creams, sun screen formulations include in addition to UV-A and UV-B filters emollients and other skin caring additives.

Emollients, also referred to as cosmetic oils, are compounds that soften and smooth the scales of the skin, which help to reduce rough and flaky skin. They are frequently occlusive agents, i.e. substances that provide a layer of protection that help prevent moisture (water) loss from the skin. Examples for emollients are silicones, such as dimethicone or cyclomethicone, vegetable oils, butters such as coca butter or shea butter, alcohols such as stearyl alcohol or cetyl alcohol, and petrolatum derivatives such a petroleum jelly or mineral oil. In sun screen formulations emollients are additionally used as solubilizer for crystalline UV filters. Due to their electrical characteristic, i.e. resistivity and permittivity, most of the emollients show a worse electrostatic sprayability. The same applies to other skin caring additives and to the UV filters, which are usually used in sun screen formulations.

Skin caring additives that differ from emollients are for example moisture agents such as humectants, occlusive agents, and agents that affect the natural moisturization mechanism of the skin. Specific examples for moisture agents known in the art are fructose, glucose, maltitol, maltose, mannitol, glycerin, glycerol polymers, inositol, lactitol, linolenic acid, but also honey, beeswax, hyaluronic acid, hydrogenated starch hydrolysate, natural moisturizing factor, acetylated lanolin and alcohol thereof, alanine, aspartic acid, barrier sphingolipids, ceramides, ceresin, collagen, collagen, amino acids, serum protein, or extracts and oils of plants, like canola oil, algae extract, aloe barbadensis, aloe-barbadensis extract, aloe barbadensis gel, avocado (*persea gratissima*) oil, Calendula officinalis extract and oil thereof, hydrogenated palm kernel oil, jojoba (*buxus chinensis*) oil, althea officinalis extract, apricot (*prunus armeniaca*) kernel oil, arnica montana extract, birch (*betula alba*) bark extract, borage (*Borago officinalis*) extract, butcherbroom (*ruscus aculeatus*) extract, candelilla (*euphorbia cerifera*) wax, canola oil, cardamon (*elettaria cardamomum*) oil, carnauba (*copernicia cerifera*) wax, carrot (*Daucus carota sativa*) oil, castor (*Ricinus communis*) oil, chamomile (*anthemis nobilis*) oil, clary (*salvia sclarea*) oil, cocoa (*Theobroma* cacao) butter, coconut (*Cocos nucifera*) oil, corn (Zea *mays)* oil, eucalyptus globulus oil, evening primrose (*Oenothera biennis*) oil, nut oils for example macadamia ternifolia nut oil, olive (Olea *europaea*) oil lavender (*Lavandula angustifolia*) oil, lemon (*citrus medica limonum*) oil, rice (*Oryza sativa*) bran oil, peppermint (*Mentha piperita*) oil, rosemary (*Rosmarinus officinalis*) oil, rose oil, safflower (*carthamus tinctorius*) oil, sage (*salvia officinalis*) oil, sandalwood (*santalum album*) oil, sesame (*Sesamum indicum*) oil, silk powder, sunflower *(Helianthus annuus)* seed oil sweet almond (*prunus amygdalus dulcis*) oil, soybean (*glycine soja*) oil, wheat (*Triticum vulgare*) germ oil, and ylang ylang (*cananga odorata*) oil, etc.

Even though some of the cosmetic formulations have appropriate electrical characteristics, it is still not possible to electrostatically spraying these formulations such that all parts of the skin are covered by the cosmetic formulation. This result into a so-called streaking effect on the skin, i.e. the applied cosmetic formulation creates a "zebra crossing" pattern on the skin.

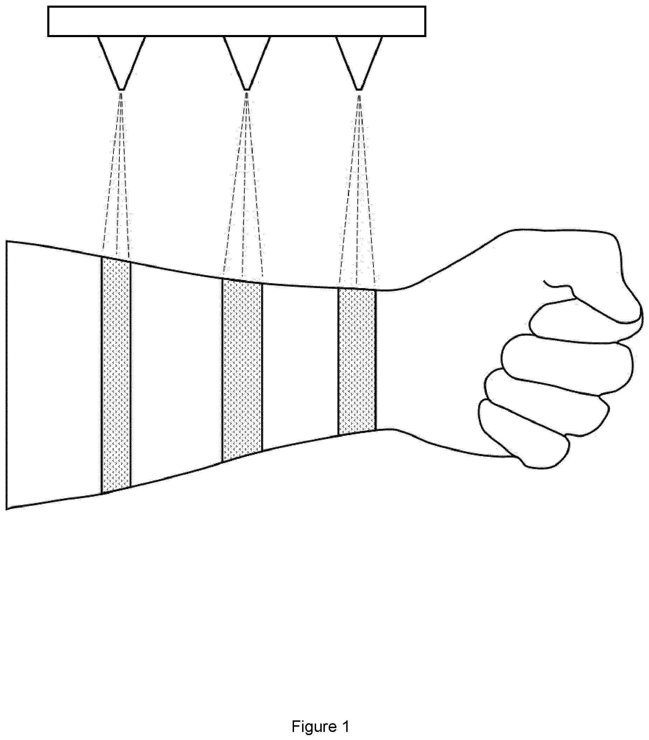

Figure 1 schematically shows the undesired "zebra crossing" pattern on the skin, which occurs if the cosmetic formulation is not uniformly sprayable and thus, no homogenous distribution of the formulation on the skin is possible.

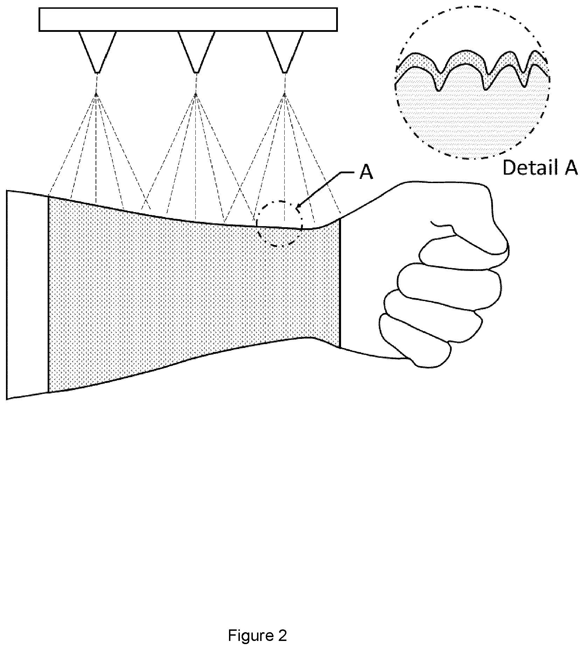

Figure 2 schematically shows the full coverage of the skin by using a cosmetic formulation of the invention, i.e. no streaking effect occurs. The enlarged section "Detail A" of Figure 6 shows a part of the skin, which is covered with the cosmetic formulation according to the invention.

In EP 19 197 645 cosmetic formulations are described, which show an improved electrostatical sprayability, i.e. the distribution of the formulation on the skin is homogenous and thus no streaking effect occurs, an improved protection against harmful UV-A and UV-B radiation, and a good water resistance. However, even though these cosmetic formulations comprises emollients, there is an interest to improve the caring effect of these formulations.

Hence, object of the application was to provide an electrostatically sprayable cosmetic formulation that has an improved caring effect, in particular a skin caring effect, by maintaining its good electrostatic sprayability, i.e. the coverage of the surface of the skin in nearly 100 %, and a good sun protection efficiency, i.e. a sun protection efficiency that is comparable to sun screen formulations known in the prior art.

It has been surprisingly found that the caring effect of the cosmetic formulation can be improved by maintaining a good electrostatic sprayability and a good sun protection efficiency, if the cosmetic formulation comprises in combination with ethanol, at 20 °C liquid polar emollients, polyalkyleneoxy terminated polyamide and acrylate as film forming agents, and UV filters, additives in relative high amounts and wherein at least one compound of the additives is a moisture agent.

Hence, according to the claimed invention, in order to ensure that the cosmetic formulation shows an improved caring effect, the additives comprise at least one compound that is a moisture agent. The moisture agent is different from the at 20 °C liquid polar emollient present in the cosmetic formulation of the invention. In other words, according to the invention the polar emollient(s) and the moisture agent(s) of the cosmetic formulation are different compounds. Additionally, the amount of additives used in the cosmetic formulation is relative high in comparison to the amount of additives usually used in cosmetic formulations, in particular used in electrostatically sprayable cosmetic formulations.

Furthermore, in order to ensure that the cosmetic formulation shows a good sprayability, i.e. no streaking effect occurs and thus a nearly 100% coverage of the skin can be achieved, the cosmetic formulation has to comprise a film forming agent mixture, which contains a polyalkyleneoxy terminated polyamide in combination with an acrylate, ethanol in a sufficient amount and liquid polar emollients, whereby the polarity of the emollients is given by their log $K_{ow}$ value.

Additionally, it has been found out that by using the film forming agent mixture according to the invention the use of a thickening agent in the cosmetic formulation is not necessary.

Therefore, the present invention provides an electrostatically sprayable cosmetic formulation, which comprises

a) 20 to 60 wt.-% ethanol;
b) 5 to 50 wt.-% emollients, which are liquid at 20 °C and have a log $K_{ow}$ of 2.0 to 10;
c) 10 to 55 wt.-% of UV-A and/or UV-B filters;
d) 0.5 to 15 wt.-% of a polymeric film forming agent mixture comprising at least one polyalkyleneoxy terminated polyamide and at least one acrylate; and
e) more than 4 wt.-% of cosmetic additives, based on the total weight of the formulation;

wherein at least one compound of the cosmetic additives is a moisture agent, the water content present in the formulation is less than 5 wt.-%, based on the total weight of the formulation, and the sum of the weight of the components is 100 %.

The cosmetic formulation as defined above shows due to the relative high content of additives, wherein at least one compound of the cosmetic additives is a moisture agent that is different from the emollients, the desired improved caring effect by maintaining its good electrostatic spraying behavior, high sun protection factor (SPF) and good water resistance.

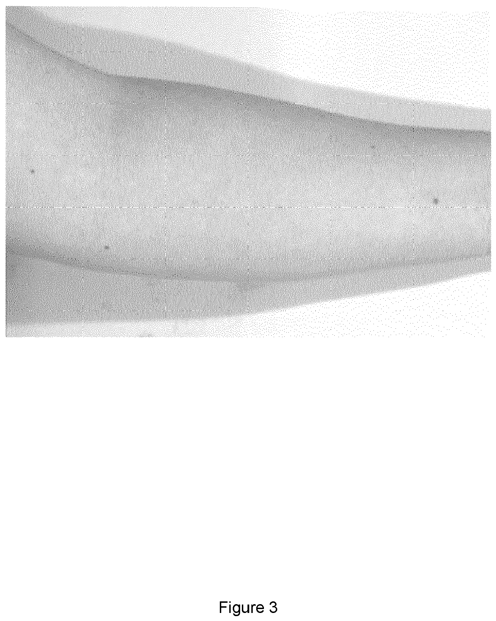

Figure 3 shows the untreated skin surface of forearms of a subject (reference measurement).

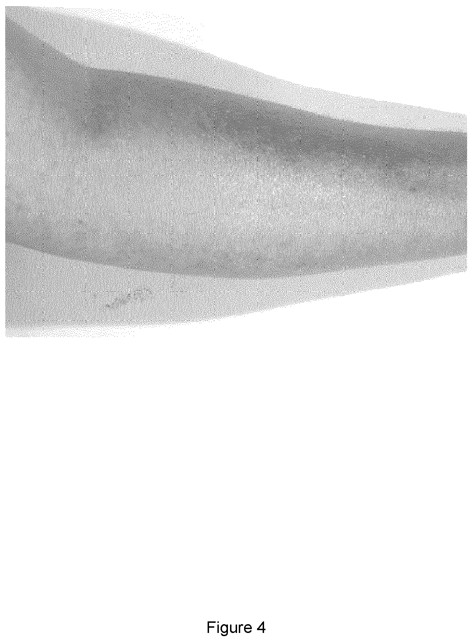

Figure 4 shows the skin surface of forearms of subjects treated with the inventive Formulation SPF 50 according to the spray test 2 as described below.

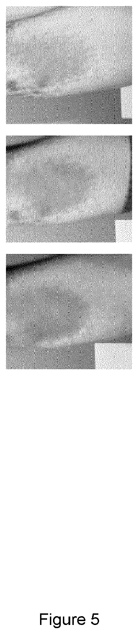

Figure 5 shows the skin surface of forearms of subjects treated with a formulation indicated as SPF 30 according to the spray test 2 as described below, whereby the formulation does not include a sufficient amount of additives according to the invention.

The abbreviation "wt.-%" or "w/w", as used herein, means "weight percentage" and refers to the weight amount of a compound in relation to the (total) weight of a formulation of compounds or of a substrate if nothing else is explicitly stated or obvious under the circumstances.

The term "comprising" includes "consisting essentially of' and also "consisting of".

In the present specification, the use of "a" in the singular also comprises the plural ("some"), and vice versa, unless the context clearly indicates the contrary.

The cosmetic formulation in the meaning of the invention is a formulation that is selected from the group consisting of sun screen, skin care, hairspray, cosmetic foundation, lipstick, hair dye, and self-tanning formulations. The cosmetic formulation can be used alone or in a mixture comprising other cosmetic formulations. Preferably, the cosmetic formulation is a sun screen formulation.

The electrostatically sprayable cosmetic formulation having the specific combination of components according to the invention preferably shows an specific electrical resistance of 15 to 100 $GOhm \cdot mm^2/m$, more preferably of 20 to 85 $GOhm \cdot mm^2/m$, it is even more preferred that the specific electrical resistance is not higher than 80, not higher than 60, not higher than 50 $GOhm \cdot mm^2/m$, most preferably the electrostatic resistance is between 35 to 48 $GOhm \cdot mm^2/m$, determined as described below (see measurement methods). This ensures that the formulation is good electrostatically sprayable and thus no undesired streaking effect on the skin occurs.

In addition, in order to provide a cosmetic formulation which shows a good electrostatic spraying behavior, the formulation preferably has a surface tension of 15 to 50 mN/m, more preferably of 20 to 45 mN/m, 22 to 40 mN/m, more preferably 25 to 35 mN/m determined by the stamp method as described below (see measurement methods).

Furthermore, the relative permittivity of the cosmetic formulation according to the invention may be at least 2.0, preferably at least 2.3, more preferably at least 2.5, most preferably at least 2.6. In particular, the relative permittivity of cosmetic formulation preferably is between 2.4 and 25.0, more preferably between 2.4 and 20.0, or between 2.4 and 10.0, most preferably between 2.4 and 5.0. The measurement method for determining the relative permittivity according to the invention is described below (see measurement methods).

Moreover, the cosmetic formulation of the invention may have a viscosity of from 0.65 mPa·s to 5000 mPa·s, preferably of from 1.0 to 1000 mPa·s, 5.0 to 500 mPa·s, more preferably of from 10.0 to 100 mPa*s, determined as described below (see measurement methods). In particular a good spraying behavior of the cosmetic formulation can be observed if the cosmetic formulation has a viscosity below 4.0 mPa·s. A cosmetic formulation having a viscosity of at least 8.0 mPa·s shows a sufficient spraying behavior.

For spaying the cosmetic formulation on the subject every electrostatic device known in the art can be used. However, it is preferred to use a device as described in DE 10 2017 108 610.2, which is further specified in DE 10 2017 108 612.9, DE 10 2017 108 613.7, DE 10 2107 108 614.5 and DE 10 2107 108 615.3.

As mentioned above, in order to ensure that the cosmetic formulation has the required electrostatic characteristics and thus shows a good electrostatic spraying behavior the formulation comprises ethanol in a sufficient amount in combination with liquid polar emollients.

It has been further found out that in addition to the sprayability of the emollients and thus of the total cosmetic formulation, also the sprayability of other components present in the cosmetic formulation can be positively influenced by the presence of ethanol in the formulation. For example, the highly viscous UV-B filter octocrylene shows a worse electrostatic sprayability in particular at low voltages. However, in case octocrylene is mixed with ethanol, the spraying quality can be improved (see results of Table 1 below). The spraying test was carried out such that the distance from a spraying nozzles to the outer left side of a metal tube was 13 cm. The pre-set voltage of three subsequent tests were 20, 30 and 40 kV, respectively. For the fluid pump a voltage of 3.7 V was used. After changing each test liquid, the tube system was rinsed with isopropanol and/or ethanol. The spraying behavior of the formulations was optically evaluated and the spraying pattern was assessed to the following scale: good; moderate, poor and nearly not sprayable.

Table 1

| Formulation | Electrical conductivity [μm/cm] (at °C) | Surface tension [mN/m] (°C) (bubble pressure method; at 20s bubble lifetime) | Spraying results | | |
|---|---|---|---|---|---|
| | | | *Pre-set 20 kV* | *Pre-set 30 kV* | *Pre-set 40 kV* |
| Octocrylene (1 %) / Ethanol (99 %) | 0.5 (25.6 °C) | n.d. | nearly no spraying at 16 kV | moderate spraying at 23 kV | moderate to good spraying at 29 kV |
| Octocrylene (10%) / Ethanol (90 %) | 0.7 (22.6 °C) | 22.34 (26.8 °C) | nearly no spraying at 16 kV | poor to moderate spraying at 23 kV | moderate to good spraying at 30 kV |
| Octocrylene (100 %) | 0.0 (at 23.7 °C) | Too viscous n.d. | nearly no spraying at 16 kV | nearly no spraying at 16 kV | poor to moderate spraying at 34 kV |
| n.d. not determined | | | | | |

As a general trend it can be seen that the spraying quality raises with the ethanol content. However, a high ethanol content in cosmetic formulations, in particular in sun screen formulations, is not desired since ethanol shows the tendency of drying out and irritating the skin, which is counterproductive in terms of the desired improved caring properties of the cosmetic formulation.

According to the invention, the amount of ethanol present in the cosmetic formulation can be reduced, in case ethanol is used in combination with emollients and the film forming agent mixture as defined in the invention. In that case, an ethanol content of 20 to 60 wt.-%, based on the total weight of the formulation, is sufficient. In particular, it is preferred that ethanol content is at least 25 wt.-%, at least 35 wt.-%, at least 40 wt.-%, more preferably at least 45 wt.-%, but not higher than 58 wt.-%, not higher than 55 wt.-%, more preferably not higher than 52 wt.-%, based on the total weight of the formulation. In a preferred embodiment of the invention, the ethanol content is between 40 and 55 wt.-%, based on the total weight of the formulation. In another preferred embodiment, the ethanol content is between 38 and 48 wt.-% or between 40 and 46 wt.-%, based on the total weight of the composition.

The emollients (cosmetic oil) of the invention are emollients which are usually used in cosmetic products such like sun screen formulations, i.e. substance that provide a layer of protection that helps prevent moisture (water) loss from the skin as described above. It is further required that the emollients are liquid at 20 °C and polar.

In the cosmetic field, one possibility to define the polarity of a compound is to determine the n-octanol/water partition coefficient ($K_{ow}$ or P). The partition-coefficient refers to the ratio of concentrations of the compounds in the mixture of these two immiscible phases at equilibrium. Hence, the partition coefficient measures how hydrophilic ("water-loving") or hydrophobic ("water-fearing") the tested compound is. In most of the cases the n-octanol/water partition coefficient is stated as decade logarithms log $K_{ow}$ or log P. The determination of the n-octanol/water partition coefficient is described for example in J. Sangster, "Octanol-Water Partition Coefficients: Fundamentals and Physical Chemistry", Vol. 2 of Wiley Series in solution chemistry, John Wiley & Sons, Chichester, 1997.

According to the invention, the log $K_{ow}$ of the emollients, which are suitable for the cosmetic formulation of the invention, is between 2.0 to 10.0, preferably between 2.5 and 9.5, 3.0 and 8.5, or between 3.5 and 7.5.

An emollient known in the prior art which fulfills the above discussed requirements is suitable for the cosmetic formulation of the invention. Examples of suitable emollients are: phenoxyethyl caprylate, PPG-3 myristyl ether, dibutyl adipate, dicaprylyl carbonate, isoamyl cocoate, diethylhexyl carbonate, isopropyl myristate, isopropyl palmitate, PPG-14 butyl ether, decyl cocoate, PPG-15 stearyl ether, C12-C15 alkyl benzoate and combinations thereof.

It is preferred that the cosmetic formulation of the invention comprises at least two but not more than 6, preferably not more than 4 different emollients. In particular, it is preferred that the cosmetic formulation comprises phenoxyethyl caprylate, dibutyl adipate, dicaprylyl carbonate and combinations thereof as emollients. In particular, it is preferred that the cosmetic formulation of the invention comprises a mixture consisting of these three emollients.

The different emollients are used in different or equal amounts in the formulation so that the total amount of emollients is between 5 to 50 wt.-%, based on the total weight of the formulation. Preferably, the total amount of emollients present in the formulation is from 8 to 40 wt.-%, 10 to 35 wt.-%, 11 to 25 wt.-%, 12 to 20 wt.-%, based on the total weight of the formulation.

The cosmetic formulation of the invention shows a sufficient sun screen protection. This property is indicated by the so-called sun protection factor (SPF). The SPF is a measure of the fraction of sunburn producing UV radiation that reach the skin. For example, "SPF 15" means that 1/15th of the burning radiation will reach the skin, assuming sunscreen is applied evenly at a thick dosage of 2 milligrams per square centimeter. The determination of the SPF of a sun screen formulation is described in detail in ISO 24444:2010 (E).

The cosmetic formulation of the invention has a SPF of at least 15, preferably of at least 20, of at least 25, of least 30, more preferably of at least 50 or higher, measured according to ISO 24444:2010 (E). Sun screen formulations which have a SPF of 60 and higher are indicated as SPF 50+ sun screen formulations.

The ability to protect the subject from harmful radiation of the sun depends inter alia on the amount of UV-A and UV-B filters present in the cosmetic formulations. In general, a mixture of UV-A and UV-B filters are used. In the present invention, the cosmetic formulation comprises UV-A and/or UV-B filters in an amount of 10 to 55 wt.-%, preferably of 12 to 50 wt.-%, 15 to 45 wt.-%, 20 to 40 wt.-%, more preferably between 16 and 42 wt.-% or between 25 and 35 wt.-%, based on the total weight of the formulation.

Moreover, with respect to a good electrostatic sprayability of the formulation, it is preferred that the UV-A and/or UV-B filters have a log $K_{ow}$ of between 2.0 to 7.0, preferably between 2.5 and 6.5, more preferably between 3.0 and 6.0.

As mentioned above, in general a mixture of UV-A and UV-B filters are used in sun screen formulations. This is also the case for the cosmetic formulation of the invention, whereby the amount of UV-A and UV-B filters can be equal or different. Preferably, the concentrations of the UV-A and B filters used in the cosmetic formulation of the invention are in accordance with the requirements according to Regulation (EC) No. 1223/2009 on cosmetic products, Annex VI.

In particular it is preferred that the cosmetic formulation comprises at least 3, at least 4 but not more than 10, preferably not more than 6 different UV-filters which can be UV-A and/or UV-B filters.

Any UV-filter known in the prior art and which does not negatively influence the electrostatic sprayability of the formulation can be used in the invention. Suitable UV filters are disclosed in Regulation (EC) No. 1223/2009 on cosmetic products, Annex VI. Some of these examples are: phenylene-1,4-bis-(2-benzimidazyl)-3,3'-5,5'-tetrasulfonacid salts; phenylbenzimidazole sulfonic acid; 2-phenylbenzimidazole-5-sulfonic acid salts; 1,4-di(2-oxo-10-sulfo-3-bornylidenemethyl) benzene and its salts; 4-(2-oxo-3-bornylidenemethyl)-benzenesulfonic acid; 2-methyl-5-(2-oxo-3-bornylidenemethyl) sulfonic acid salts; 2,2'-methylene-bis- (6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]-disiloxanyl] propyl] phenol; 3-(4-methylbenzylidene)-camphor; ethylhexyl salicylate; terephthalidendicamphersulfonic acid; 4-(dimethylamino) benzoic acid (2-ethylhexyl) ester; 4-(dimethylamino) benzoic acid amyl ester; 4-methoxybenzalmalon-di(2-ethylhexyl) ester; 4-methoxycinnamate (2-ethylhexyl) ester, 4-methoxycinnamate; 2-hydroxy-4-methoxybenzophenone; 2-hydroxy-4-methoxy-4'-methylbenzophenone; 2,2'-dihydroxy-4-methoxybenzophenone; hexyl-2-(4'-diethylamino-2'-hydroxybenzoyl) benzoate 4-(tert-butyl)-4'-methoxydibenzoylmethane; homomethylsalicylate (INCI: Homosalate); 2-ethylhexyl-2-hydroxybenzoate; 2-ethylhexyl-2-cyano-3,3-diphenylacrylate (INCI: Octocrylene); dimethicodiethylbenzalmalonate; 3-(4-(2,2-ethoxycarbonyl-vinyl)-phenoxy)-propenyl)-methoxy-siloxane/dimethylsiloxane copolymer; 2,4-bis-{[4-(2-ethyl-hexyloxy)-2-hydroxy]-phenyl}-6-(4-methoxyphenyl)-1,3,5-triazine (INCI: bis-ethylhexyloxyphenol methoxyphenyl triazine); dioctylbutylamido triazone (INCI: diethylhexyl-butamido triazone); 2,4-bis-[5-1-(dimethylpropyl)benzoxazol-2-yl-(4-phenyl)-imino]-6-(2-ethylhexyl)-imino-1,3,5-triazine having the (CAS No 288254-16-0); ethylhexyl triazine; 4,4',4"-(1,3,5-triazine-2,4,6-triyltriimino)-tris-benzoic acid-tris-2-ethylhexyl ester (also: 2,4,6-tris[anilino-(p-carbo-2'-ethyl-1'-hexyloxy)]-1,3,5-triazine (INCI: ethylhexyl triazone); 2,4,6-tribiphenyl-4-yl-1,3,5-triazine; butyl-methoxydibenzoylmethane; isoamyl-p-methoxycinnamate and diethylamino hydroxybenzoyl hexyl benzoate.

Preferably, the UV-filters are selected from the group consisting of homosalate, butyl methoxydibenzoylmethane, octocrylene, diethylhexyl butamido triazone, ethylhexyl salicylate, bis-ethylhexyloxyphenol methoxyphenyl triazine, diethylamino hydroxybenzoyl hexyl benzoate and combinations thereof.

In particular, it is preferred that the cosmetic formulation comprises a mixture of UV-A and UV-B filters which consists of butyl methoxydibenzoylmethane, octocrylene, ethylhexyl salicylate and homosalate.

In addition to the specific combinations of ethanol, emollients and UV-A/UV-B filters as described above, according to the invention, in order to ensure the sprayability of the cosmetic formulation the cosmetic formulation has to comprise a film forming agent mixture that comprises at least one polyalkyleneoxy terminated polyamide and at least one acrylate. Furthermore, the use of the film forming agent mixture according to the invention allows the SPF to be increased without the need of increasing the amount of UV filters present in the formulation and thus to maintain the good sprayability of the formulation.

Every known polyalkyleneoxy terminated polyamide, as for example described in US 2014/0212363, can be used in the present invention. However, it is preferred that the polyalkyleneoxy terminated polyamide is a condensation product of dilinoleic acid, ethylenediamine and polypropylene glycol diamine end-capped with polyethylene glycol-poylpropylene glycol (PEG-PPG)-32/10 aminopropyl methyl ether. It is more preferred that the polyalkyleneoxy terminated polyamide is selected from the group consisting of a polyamide-3, a polyamide-4, and mixtures thereof, such as OleoCraft MP-30, OleoCraft MP-32, OleoCraft HP-31, OleoCraft HP-33, SYLVASOL 80, SYLVACLEAR AF1900V, SYLVACLEAR PE1800V, SYLVACELEAR PA1200V and SYLVACLEAR WF1500V available for instance from Arizona Chemical or Croda. Preferably, the polyalkyleneoxy terminated polyamide is polyamide-3.

Furthermore, although, according to the invention, the acrylate can be any one known in the art, such as for example acrylate/dimethicone copolymer, styrene acrylate copolymer or vinyl acetate (VA)/butyl maleate/isobornyl acrylate copolymer, it is preferred that the acrylate is an acrylate/amide copolymer. More preferably, the acrylate/amide copolymer is an acrylate/amide copolymer with alkyl groups having 6 to 16 carbon atoms. Most preferably, the acrylate/amide copolymer is an acrylates/octylacrylamide copolymer. This polymer comprises carboxyl groups attached to the polymer main chain and octyl side chains and is commercially available, for example under the trademark DERMACRYL 2.0 or DERMARYL 79 from AkzoNobel, DE.

According to the invention, in addition to the polyalkyleneoxy terminated polyamide and the acrylate, the film forming agent mixture may contain as film forming agents monomer, copolymers, cross polymers and terpolymer of organ-silicone hybrid, biopolymer, abietic acid derivatives, polyolefins, silicone resins as known in the prior art, in particular vinyl acetates, maleates, alkyl esters, long chain and short chain carboxylic acids, polyamides, ethanol (and) crotonic acid/vinyl $C_{8-12}$ esters/VA/bis-vinyldimethicone crosspolymer, isododence (and) acrylate/dimethicone copolymer, acrylates/dimethicone copolymer (and) cyclopentasiloxane, methyl dihydroabietate, hydrogenated polycyclopentadiene isoalkyl (and) isododecane, vinylpyrollidone (VP)/hexadecane copolymer hydrogenated dimer dilinoleyl/diemethylcarbonate copolymer, trimethylpentanediol/adipic acid/glycerin crosspolymer, or combinations thereof.

As mentioned above, the cosmetic formulation of the invention comprises the polymeric film forming agent mixture in an amount of 0.5 to 15 wt.-%, based on the total weight of the formulation; preferably, in an amount of between 0.7 and 13 wt.-%, between 1 and 10 wt.-%, between 2 and 8 wt.-%, between 3 and 7 wt.-% and more preferably between 4 and 6 wt.-%, based on the total weight of the formulation.

Furthermore, according to the invention, the amount of the polyalkyleneoxy terminated polyamide and the amount of the acrylate present in the film forming agent mixture can be equal or different.

In particular, the cosmetic formulation comprises the polyalkyleneoxy terminated polyamide in an amount of 0.5 to 10 wt.-%, preferably in an amount of 1 to 8 wt.-%, more preferably in an amount of 2 to 6 wt.-%, based on the total weight of the formulation. The same amounts are suitable for the acrylate present in the composition as film forming agent.

Most preferably, the cosmetic formulation comprises the polyalkyleneoxy terminated polyamide and the acrylate in equal amounts, preferably each component in an amount of 2 wt.-%, based on based on the total weight of the formulation.

According to the invention, in addition to the good sprayability the cosmetic formulation of the invention provides an improved caring effect.

The increase of the water content of the skin (skin hydration) is a crucial factor regarding the extent of caring effect of a cosmetic formulation. The skin hydration can be determined by the so-called Corneometer method as described in detailed in paragraph "Measurement methods" below. In this method, the fact is used that water differs markedly from most substances as far as its dielectric constant is concerned. A quantitative proof of changes to the water content of the skin can thus be achieved by means of capacity measurements in a non-invasive manner. Depending on the water content a measuring capacitor reacts to the capacity changes of the samples. These capacity changes are registered by the measuring head capacitor and the data are processed automatically by the Corneometer to a digital measured value. The values measured by the Corneometer method specify the degree of moisture of the skin surface, i.e. the difference of water content before and after skin treatment with the cosmetic formulation. The numbers given by the Corneometer are no absolute value and may be varied depending on the Corneometer used for measurement. However, Corneometer changes which are ascertained by a relative examination do not depend on the type of Corneometer. Thus, according to the invention, not percentage changes, but changes in Corneometer Units [C.U.] are analyzed.

The C.U. values of the invention refer to the difference of the initial value, i.e. the skin hydration value before the application of the cosmetic formulation (initial value), and the value measured within a certain time period after application of the cosmetic formulation. A C.U. value between 0.5 to 2, preferably between 1 and 1.8 within a certain time period after application of the cosmetic formulation indicates that no increase in skin hydration is obtained. The use of the cosmetic formulation of the invention results into a C.U. value of at least 8, preferably of at least 10, at least 15, more preferably of at least 18 within one hour after single application of the formulation to the skin. In one embodiment of the invention, the C.U. value is not higher than 40, not higher than 35, or not higher than 25, within one hour after single application of the cosmetic formulation. It is also preferred that the C.U. value within a time period of 6 hours after the single application of the cosmetic formulation to the skin is constant. Additionally, it is preferred that after 6 hours of the single application the C.U. value is about 15, about 18 or more preferred about 20. Additionally, it is preferred that after 24 hours of the application of the cosmetic formulation the C.U. is between 2 and 8, more preferably between 3 and 5.

In order to ensure that the cosmetic formulation shows a good, preferably an improved caring effect, in particular that the skin hydration is increased, the cosmetic formulation of the invention comprises a relative high amount of cosmetic additives in comparison to the amount of additives usually present in electrostatically sprayable cosmetic formulation.

According to the invention, the relative high amount of cosmetic additives is more than 4.0 wt.-%, preferably more than 4.05, more than 4.2 wt.-%, more than 5.0, more than 5.5 wt.-%, or more than 6.0 wt.-% but not higher than 8.0 wt.-%, preferably not higher than 7.0 wt.-%, based on the total weight of the cosmetic formulation.

In particular it is preferred, that the amount of cosmetic additive present in the cosmetic formulation of the invention is between 4.05 and 7.5 wt.-%, 4.1 and 7.0 wt.-%, or more preferred between 4.1 and 6.8 wt.-%, based on the total weight of the formulation.

The cosmetic additives according to the invention comprises at least one moisture agent, preferable at least two various moisture agents, which differ from each other and the polar emollients as described above. Preferably, the cosmetic additives comprises three various moisture agents that differ from each other and the emollients used in the cosmetic formulation.

Suitable moisture agents that can be used in the cosmetic formulation of the present invention are described above. However, it is preferred the moisture agents are selected from the group consisting of fructose, glucose, glycerin, glycerol polymers, linolenic acid, hyaluronic acid, beeswax, hydrogenated starch hydrolysate, acetylate lanolin alcohol, ceramides, ceresin, collagen, collagen, algae extract, sweet almond oil, aloe barbadensis, aloe-barbadensis extract, aloe barbadensis gel, avocado oil, canola oil, *Calendula officinalis* extract, hydrogenated palm kernel oil, and jojoba oil.

More preferably, the moisture agents are selected from the group consisting of glycerin, aloe barbadensis, aloe-barbadensis extract, aloe barbadensis gel, jojoba oil and mixtures thereof.

In a further preferred embodiment of the invention the cosmetic additives comprises in any case a mixture of aloe-barbadensis extract and jojoba oil.

Furthermore, according to the invention, it is preferred that the amount of each of the above listed moisture agents present in the cosmetic formulation is between 0.5 and 3.5 wt.-%, between 1.0 and 2.5 wt.-%, more preferably between 1.5 and 2.0 wt.-%, based on the total weight of the cosmetic formulation.

Additionally, it is preferred that the cosmetic additives and thus the cosmetic formulation of the invention comprises in addition to the above mentioned moisture agents further compounds such as cosmetically acceptable carriers, oils, sterols, amino acids, powders, colorants, pigments, dyes, pH adjusters, perfumes, essential oils, vitamins E, pro-vitamins, essential fatty acid, sphingolipids, self-tanning compounds, etc. and combinations thereof.

Preferably, according to the invention, the additives are selected from the group consisting of perfumes, vitamins and/or pro-vitamins such as tocopherol and/or tocopheryl acetate, and combinations thereof.

Furthermore, the amount of water present in the cosmetic formulation of the invention is less than 5 wt.-%, preferably less than 3 wt.-%, less than 1 wt.-%, more preferably less than 0.1 wt.-%, based on the total weight of the formulation. It is preferred that the water content of the formulation of the invention is not higher than 2.5 wt.-%, more preferably not higher than 0.5 wt.-%. In particular, it is preferred that the cosmetic formulation of the invention is water free, i.e. the water content of the cosmetic formulation of the invention is zero. According to the invention, the term "water free" includes that each compound used in the cosmetic formulation contains no residues of water, for example it is preferred that denaturized ethanol, which does not contain water, and water free glycerin (Glycerin Ph. Eur. 99%), is used in the cosmetic formulation of the invention.

Furthermore, since the compounds used in the cosmetic formulation of the invention, in particular the active compounds such as UV-filters, are completely soluble in the formulation of the invention, the formulation of the invention has an improved stability, in particular with respect to storage, and thus there is no need to use thickening agents as described for example in WO 01/12139 in the cosmetic formulation of the invention. Thickening agents are for example silicones, waxes, clays, silicas, salts, natural and synthetic esters, fatty alcohols, and mixture thereof.

In a further embodiment of the invention the electrostatically sprayable cosmetic formulation comprises

a) 35 to 55 wt.-% ethanol;
b) 8 to 20 wt.-% emollients, which are liquid at 20 °C and have a log $K_{ow}$ of 2.0 to 10;
c) 16 to 45 wt.-% of UV-A/UV-B filter mixture;
d) 2 to 5 wt.-% of a polymeric film forming agent mixture comprising at least one polyalkyleneoxy terminated polyamide and at least one acrylate; and
e) 4.1 to 7.0 wt.-% of cosmetic additives, based on the total weight of the formulation;

wherein at least one compound of the cosmetic additives is a moisture agent, which is selected from the group consisting of glycerin, aloe barbadensis, aloe-barbadensis extract, aloe barbadensis gel, and jojoba oil; the water content present in the formulation is less than 1 wt.-%, based on the total weight of the formulation; and the sum of the weight of the components is 100 %.

In a further preferred embodiment of the invention, the electrostatically sprayable cosmetic formulation comprises

a) 35 to 55 wt.-% ethanol;

b) 8 to 20 wt.-% emollient mixture consisting of phenoxyethyl caprylate, dibutyl adipate and dicapryic carbonate;

c) 16 to 45 wt.-% of UV-A/UV-B filter mixture consisting of butyl methoxydibenzylmethane, octocrylene, ethylhexyl salicylate and homosalate;

d) 2 to 5 wt.-% of a polymeric film forming agent mixture consisting polyamide 3 and acrylate/octylacrylamide copolymer; and

e) 4.1 to 7.0 wt.-% of cosmetic additives, based on the total weight of the formulation;

wherein the cosmetic additives comprises a mixture of at least two moisture agents selected from the group consisting of glycerin, aloe barbadensis, aloe-barbadensis extract, aloe barbadensis gel and jojoba oil; the water content present in the formulation is less than 1 wt.-%, based on the total weight of the formulation; and the sum of the weight of the components is 100 %.

In an even more preferred embodiment of the invention, the cosmetic formulation consists of the mixtures of compounds as defined above and the cosmetic formulation is water free.

In particular, it is preferred that the electrostatically sprayable cosmetic formulation consists of

a) 40 to 50 wt.-% ethanol;

b) 10 to 14 wt.-% emollient mixture consisting of 3.0 to 4.5 wt.-% of phenoxyethyl caprylate, 3.5 to 5.0 wt.-% adibutyl adipate and 3.5 to 4.5 wt-% dicapryic carbonate;

c) 25 to 42 wt.-% of UV-A/UV-B filter mixture consisting of 2.0 to 4.0 wt.-% butyl methoxydibenzylmethane, 8.0 to 12.0 wt.-% octocrylene, 3.0 to 8.0 wt.-% ethylhexyl salicylate and 12.0 to 18.0 wt.-% homosalate;

d) 2 to 5 wt.-% of a polymeric film forming agent mixture consisting of 1 to 2.5 wt.-% of polyamide 3 and 1 to 2.5 wt.-% of acrylate/octylacrylamide copolymer; and

e) 4.1 to 6.75 wt.-% of cosmetic additive mixture consisting of 1.8 to 2.5 wt.-% glycerin, 0.8 to 1.2 wt.-% aloe-barbadensis extract, 0.8 to 1.2 wt.-% jojoba oil, 0.1 to 0.25 wt.-% tocopherol, 0.3 to 0.8 wt.-% tocopherol acetate and 0.3 to 0.8 wt.-% perfume, based on the total weight of the formulation;

wherein the cosmetic formulation is water free and the sum of the weight of the components is 100 %.

The water resistance of the cosmetic formulation according to the invention is preferably above 60 %, more preferably above 70 %, even more preferably above 80%, most preferably above 90 %, as usually determined and described below (see measurement methods).

The examples that follow are intended for illustrating the invention in more detail.

## Examples

### 1. Measurement methods

#### 1.1 Specific electrical resistance

The specific electrical resistance was determined by the means of an Electrospray Paint Test Meter of DCA Electronic Ltd. The measuring current was between 85 μA and 100 μA or higher. The measuring range was 10 kOhm up to 5 MOhm. The measured electrical resistance was approximately conversed to the specific electrical resistance according to ASTDM D5682-08.

#### 1.2 Surface tension

The surface tensions of the formulations as described herein were measured by two different measurement methods, the stamp method and the bubble pressure method. Due to the low viscosity of the cosmetic formulations according to the invention, the stamp method was used to determine the surface tension of these cosmetic formulations.

#### 1.2.1 Stamp method

The metal stamp can be used to determine the surface tension of liquids and highly viscous media (e.g. polymer melt or adhesives). The liquid is applied to the stamp, which forms an entire surface and a drop on the stamp. The stamp material has no influence on the measurement result.

The surface tension of the liquid can be calculated from the drop contour and the density of the liquid using the SCA 20 software (www.dataphysics-instruments.com, DATAPhysics OCA20) and the Young-Laplace equation.

In addition to the surface tension, the contact angle and the volume of the drop are also measured. The determined surface tension is independent of the contact angle. The same surface tension values are measured both when the droplet is enlarged and when it is reduced by suction.

*1.2.2 Bubble pressure method*

With the bubble pressure method the dynamic surface tension of the formulations in liquids can be determined. This method is carried out by using the tensiometer SITA pro line t15 (www.sita-messtechnik.de), wherein an air stream is lead into the sample liquid through a robust capillary and the pressure development necessary for the bubble generation is measured at room temperature (automatic mode: bubble lifetime from 0.02 to 20 s. It is preferred to indicate the surface tension s [mN/m] at a bubble lifetime of 20 s.

1.3 Relative permittivity

The relative permittivity was determined by the means of a High Frequency Liquid Dielectric Constant Meter (Time Domain Reflection) "ALPHA TDR-5000" of Zadow Electronics (ww.zadow-electronics.de) having two parallel wire sensors that have a length of 5 cm. The measurement method was carried out such that the TDR-500 periodically sends voltage pulses with times (250 ps) onto the sensor cable to determine the dielectric constant (K) of the formulations:

$$K = 15^2 \times (\frac{t}{r})^2$$

t = running time in ns; the running time was 5 ns    r = length of the wire sensor (5 cm)

The relative permittivity of the formulation was calculated on the basis of the determined dielectric constant.

1.4 Electrical conductivity

The electrical conductivity was measured by means of Sartorious PT-20, hand-held conductivity meter, measuring cell PY-CL1 (www.sartorius.de). Measurement at room temperature.

1.5 Viscosity

The viscosity was determined by means of a Universal Dynamic spectrometer "Physica UDS 200" at shear rates of 1 to 3000 s$^{-1}$ and 22 °C.

1.6 Spraying test 1

In this spraying test, the sprayability of cosmetic formulations to provide a homogenous distribution on the skin surface was tested.

The conditions for all test setups were the same. The tested cosmetic formulations were sprayed by using an IONIQ spraying system, which is described in detail in DE 10 2017 108 610.2, DE 10 2017 108 612.9, DE 10 2017 108 613.7, DE 10 2107 108 614.5 and.DE 10 2107 108 615.3. The tested cosmetic formulations were sprayed on the forearms of 3 and 4, respectively, subjects. The spraying time was 0.75 s (scale 1.5) and the voltage of the pump for spraying was 2.6 V. After spraying the IONIQ system was cleaned with denaturized alcohol. The formulations were leak to soak for 30 minutes without further treatment or massage. Afterwards the homogenous distribution of the formulations on the skin was determined *in vivo* by laser scanning microscopy (LSM). The tested cosmetic formulations contained fluorescein and thus by illumination at 488 nm the fluorescence of the fluorescein could be made visible. A mosaic measuring 4 x 4 mm on the skin surface was recorded at each measuring point.

### 1.7 Spraying test 2

In this spraying test, also the IONIQ spray system was used and the conditions for all test setups were the same. The tested cosmetic formulations were sprayed on the forearm of a subject and were leak to soak without any further treatment. After 1.5 and 30 minutes the distribution of the formulations on the skin was photographed with a UV camera. Afterwards, the uniformity of the dark coloring was assessed.

### 1.8 Sun Protection Factor (SPF)

The SPF was determined according to the International standard ISO 24444:2010(E); all measurement conditions as indicated in this International standard were considered.

### 1.9 Water Resistance (Screening)

For the determination of water resistance of the formulation the SPF testing according to ISO 24444:2010 (E) was carried out twice at each subject. On one side of the back of the subject an SPF measurement (screening) according to ISO 24444:2010 (E), as described above, is carried out, whereas on the contra lateral side SPF testing is combined with a standardized watering procedure. Watering of the test area takes place in accordance with the COLIPA Guidelines of Evaluating Sun Product Water Resistance 2005.

### 1.10 Assessment of Skin Hydration

The skin hydration was determined by using a Corneometer CM 825 hydrometer with MDD4 (Multi Display Device, Courage + Khazaka electronic GmbH).

The face of the sensor was coated with a special glass, which allowed axial movement and it had a range of at least 3 mm. The principle of measurement requires that the surface of the measuring device sits evenly at a constant pressure on the skin. To ensure that this is as reproducible as possible, the face of the measuring head was very small with an area of $7x7$ mm$^2$. The inner movable part - the active face- was pressed onto the skin with a pressure of 3.5 N by means of a spring. This guarantees standardized measurements, irrespective of the investigator. During measurement, the measuring head was pressed onto the required part of the skin. After one second the measured value was recorded and processed by the computer. The value measured by the Corneometer specifies the degree of moisture of the skin surface, e.g. the difference before and after skin treatments with the cosmetic formulation.

The studies included 10 healthy volunteers. The volar forearms were used as test areas. A maximum number of three areas were available on each volar forearm. The test areas were permuted to compensate for any differences in skin.

During three days before study start the volunteers washed their volar forearms with Sodium Lauryl Ether Sulphate (10 % SLES, 4 % NaCl in water) twice daily as follows: 1. Lathering of forearms, 2. Waiting for 1 minute, 3. Washing-up with clear water. Further cosmetics like cleansing or care products were not allowed to use three days before and during the study. 45 minutes before and during each measurement, the volunteers were acclimatized at 22 °C ($\pm$ 1 °C) room temperature and 50 % ($\pm$ 5 %) relative humidity. With this procedure it was possible to standardized the skin humidity influenced by short-time conditions depending on the volunteers' individual way of appearing at the institute, e.g. by bicycle or car etc. Before the test formulations were applied to the test areas, a Corneometer measurement (ten individual values) was taken on each test area, in order to document the initial situation (t0). After taking initial values, the test formulations were applied once (2 mg/cm$^2$) on the assigned test areas by a technician. As reference an untreated area and an area treated with a positive standard were used. Subjects were advised not to treat the test areas in any way (including washing) until final measurement time. Corneometer measurements were carried out at defined intervals during a defined time period (t0; t1h; t2h; t3h; t4h; t5h; t6h; and t24h).

## 2. Cosmetic Formulations

All used raw materials are cosmetic qualities and commercially available. The used ethanol was denaturized quality (denaturant: isopropyl and tert.-butanol; Ethanol 96%), the used glycerin was Glycerin Ph. Eur. 99.8%, the used aloe barbadensis extract was aloe barbadensis herbasol extract IPM 201100.02.2 from Lipoid GmbH and the jojobal oil was Ewanol Jo from Wagner. These compounds are widely used in cosmetic products, which should be water free.

The components of each formulation were mixed together such that a homogeneous mixture of the formulation was obtained.

The cosmetic formulation according to the invention is indicated as Formulation SPF 50. The comparative formulation indicated as Formulation SPF 30 is a formulation according to EP 19 197 645.

The compositions of the formulations are given in Table 2 below.

| Material | INCI (EU) | SPF 50 (inventive) [wt.-%] | SPF30 (reference) [wt.-%] |
|---|---|---|---|
| Eusolex 9020 | BUTYL METHOXYDIBENZOYLMETHANE | 3 | 5 |
| Neo Heliopan 303 | OCTOCRYLENE | 10 | 10 |
| Neo Heliopan OS | ETHYLHEXYL SALICYLATE | 5 | 5 |
| Eusolex HMS | HOMOSALATE | 15 | - |
| Uvasorb HEB | DIETHYLHEXYL BUTSMIDO TRIAZONE | - | 3 |
| Parsol Shield | BISETHYLHEXYLOXYPHENOL METHOXYPHENYL TRIAZINE | - | 1.5 |
| Total amount of UV filters | | 33 | 24.5 |
| Tegosoft XC | PHENOXYETHYLCAPRYLATE | 3.6 | 11.6 |
| Cetiol B | DIBUTYL ADIPATE | 4.2 | 5.0 |
| Cetiol CC | DICAPRYLYL CARBONATE, TOCOPHEROL | 4.0 | 5.2 |
| Tegosoft APM | PPG-3 MYRISTYL ETHER | - | 1.0 |
| Total amount of emollients | | 11.8 | 22.8 |
| OleoCraft HP-31 | POLYAMIDE-3 | 2 | 3.0 |
| Dermacryl 79 | ACRYLATES/OCTYLACRYLAMIDE COPOLYMER | 2 | 2 |
| Total amount of film forming agents | | 4 | 3.5 |
| Ethanol | ALCOHOL DENAT. | 46 | 45.5 |
| Glycerin | GLYCERIN | 2 | 2 |
| Perfume | | 0.5 | 0.5 |
| DL-alpha-Tocopherol | TOCOPHEROL | 0.2 | 0.2 |
| DL-Alpha-Tocopherolacetate | TOCOPHERYL ACETATE | 0.5 | 0.5 |
| Aloe barbadensis herbasol extract | ALEO BARBADENSIS LEAF EXTRACT | 1.0 | - |
| Ewanol JO (jojoba oil) | SIMMONDSIA CHINENSIS SEED OIL | 1.0 | - |
| Total amount of additives | | 5.2 | 3.2 |

Table 2

*3. Results*

3.1 Spraying test 1

Spraying test 1 was carried out with the cosmetic formulations according to the invention (SPF50) and the comparative formulation (SPF 30).

Both formulations were electrostatically sprayable.

3.2 Spraying test 2

Figure 4 shows the spraying results of the Formulation SPF 50 according to the invention.

Figure 5 shows the spraying result of the Formulation SPF 30.

The UVA images clearly show that both formulations provide a homogeneous distribution on the skin.

3.3 Sun protection factor

The SPF of the Formulation according to the invention and of the formulation of comparative example was determined. The Formulation of the invention has an SPF of 51.50, the formulation of the comparative example has a SPF of 39.5.

3.4 Water Resistance

The Formulation of the invention shows as similar (sufficient) water resistance as the Formulation SPF 30.

3.5 Relative Permittivity, specific electrical resistance and surface tension

The relative permittivity, the specific electrical resistance and the surface tension (stamp method) of the Formulation of the invention were in the same ranges as of the Formulations SPF 50, 30 and 15 disclosed in EP 19 197 645.

3.6 Skin Hydration

The C.U. values of the Formulation of the invention determined after 1 hour, 6 hours and 24 hours of its application demonstrate that the use of the Formulation of the invention results into an increase of skin hydration.

3.7 Conclusion

Although the cosmetic formulation includes moisture agents in relative high amounts that show worse electrostatic sprayability properties, in order to obtain in combination with ethanol, polar emollients, UV-filters and film forming agents according to the invention the electrostatic sprayability is comparable to an electrostatically sprayable cosmetic formulation as known in the prior art.

**Claims**

1.  Electrostatically sprayable cosmetic formulation, comprising

    a) 20 to 60 wt.-% ethanol;
    b) 5 to 50 wt.-% emollients, which are liquid at 20 °C and have a log $K_{ow}$ of 2.0 to 10;
    c) 10 to 55 wt.-% of UV-A and/or UV-B filters;
    d) 0.5 to 15 wt.-% of a polymeric film forming agent mixture comprising at least one polyalkyleneoxy terminated polyamide and at least one acrylate; and
    e) more than 4 wt.-% of cosmetic additives, based on the total weight of the formulation;

    wherein at least one compound of the cosmetic additives is a moisture agent, the water content present in the formulation is less than 5 wt.-%, based on the total weight of the formulation, and the sum of the weight of the components is 100 %.

2.  Electrostatically sprayable cosmetic formulation according to claim 1, wherein the moisture agent is present in the cosmetic formulation in an amount of 0.5 to 3 wt.-%, based on the total weight of the formulation.

3. Electrostatically sprayable cosmetic formulation according to claims 1 and 2, wherein the composition does not comprise a thickening agent.

4. Electrostatically sprayable cosmetic formulation according to any one of the previous claims, wherein the cosmetic formulation comprises at least two different emollients.

5. Electrostatically sprayable cosmetic formulation according to any one of the previous claims, wherein the emollients are selected form the group consisting of phenoxyethyl caprylate, PPG-3 myristyl ether, dibutyl adipate, dicaprylyl carbonate, isoamyl cocoate, diethylhexyl carbonate, isopropyl myristate, isopropyl palmitate, PPG-14 butyl ether, decyl cocoate, PPG-15 stearyl ether, $C_{12}$-$C_{15}$ alkyl benzoate and combinations thereof.

6. Electrostatically sprayable cosmetic formulation according to any one of the previous claims, wherein the UV-A and/or UV-B filters having log $K_{ow}$ of 2.0 to 7.0.

7. Electrostatic sprayable cosmetic formulation according to any one of the previous claims, wherein the UV-A and/or UV-B filters are selected from the group consisting of butyl methoxydibenzoylmethane, octocrylene, diethylhexyl butamido triazone, ethylhexyl salicylate, bis-ethylhexyloxyphenol methoxyphenyl triazine, diethylamino hydroxybenzoyl hexyl benzoate, homosalate and combinations thereof.

8. Electrostatically sprayable cosmetic formulation according to any one of the previous claims, wherein the polyalkyleneoxy terminated polyamide is a condensation product of dilinoleic acid, ethylenediamine and polypropylene glycol diamine end-capped with polyethylene glycol-poylpropylene glycol (PEG-PPG)-32/10 aminopropyl methyl ether.

9. Electrostatically sprayable cosmetic formulation according to any one of the previous claims, wherein the acrylate is an acrylate/amide copolymer.

10. Electrostatic sprayable cosmetic formulation according to any one of the previous claims, wherein at least two compounds of the cosmetic additives are moisture agents, which differ from each other.

11. Electrostatic sprayable cosmetic formulation according to any one of the previous claims, wherein the moisture agent is selected from the group consisting of glycerin, aloe barbadensis, aloe-barbadensis extract, aloe barbadensis gel, and jojoba oil.

12. Electrostatically sprayable cosmetic formulation according to any one of the previous claims, wherein cosmetic formulation comprises in addition to at least one moisture agent as cosmetic additive compounds selected from the group consisting of perfumes, vitamin, pro-vitamins and combinations thereof as additives.

13. Electrostatically sprayable cosmetic formulation according to any one of the previous claims, wherein the cosmetic formulation is a sun screen formulation.

14. Electrostatically sprayable cosmetic formulation according to any one of the previous claims, wherein the cosmetic formulation comprises

    a) 35 to 55 wt.-% ethanol;
    b) 8 to 20 wt.-% emollients, which are liquid at 20 °C and have a log $K_{ow}$ of 2.0 to 10;
    c) 16 to 45 wt.-% of UV-A/UV-B filter mixture;
    d) 2 to 5 wt.-% of a polymeric film forming agent mixture comprising at least one polyalkyleneoxy terminated polyamide and at least one acrylate; and
    e) 4.1 to 7.0 wt.-% of cosmetic additives, based on the total weight of the formulation;

    wherein at least one compound of the cosmetic additives is a moisture agent selected from the group consisting of glycerin, aloe barbadensis, aloe-barbadensis extract, aloe barbadensis gel, and jojoba oil; the water content present in the formulation is less than 1 wt.-%, based on the total weight of the formulation; and the sum of the weight of the components is 100 %.

15. The use of a film forming agent mixture comprising at least one polyalkyleneoxy terminated polyamide and at least one acrylate, in a cosmetic formulation comprising

    a) 20 to 55 wt.-% ethanol;
    b) 8 to 50 wt.-% emollients, which are liquid at 20 °C and have a log $K_{ow}$ of 2.0 to 10;
    c) 10 to 55 wt.-% of UV-A and/or UV-B filters;
    d) 0.5 to 15 wt.-% of a polymeric film forming agent mixture comprising at least one polyalkyleneoxy terminated polyamide and at least one acrylate; and
    e) more than 4 wt.-% of cosmetic additives, based on the total weight of the formulation;

wherein at least one compound of the cosmetic additives is a moisture agent, the water content present in the formulation is less than 5 wt.-%, based on the total weight of the formulation, and the sum of the weight of the components is 100 %,
for electrostatic spraying of the cosmetic formulation.

Figure 1

Figure 2

Figure 3

Figure 4

Figure 5

| | Europäisches Patentamt<br>European Patent Office<br>Office européen des brevets | **EUROPEAN SEARCH REPORT** | Application Number<br>EP 20 21 4423 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANONYMOUS ED - DARL KUHN: "Gelled Ethanol Sunscreen and Ethanol Based Continuous Spray Sunscreen Formulary Information", IP.COM, IP.COM INC., WEST HENRIETTA, NY, US, 13 July 2011 (2011-07-13), XP013147464, ISSN: 1533-0001 | 1-3,5-14 | INV.<br>A61Q17/04<br>A61K8/34<br>A61K8/88<br>A61K8/90 |
| Y | * Ethanol based continuous spray, SPF 35; page 2 * | 1-15 | |
| | ----- | | |
| Y | DE 10 2013 215831 A1 (BEIERSDORF AG [DE]) 12 February 2015 (2015-02-12) * examples * * claims * | 1-15 | |
| | ----- | | |
| Y,D | US 2014/212363 A1 (HARMAN NANCY W [US] ET AL) 31 July 2014 (2014-07-31) * examples * * claims * | 1-15 | |
| | ----- | | TECHNICAL FIELDS SEARCHED (IPC) |
| Y | "Suncare compositions (13) ED - Darl Kuhn", IP.COM, IP.COM INC., WEST HENRIETTA, NY, US, 24 May 2017 (2017-05-24), XP013175041, ISSN: 1533-0001 * Dry touch clear gel SPF 50; page 20 * | 1-15 | A61Q |
| | ----- | | |
| | -/-- | | |

The present search report has been drawn up for all claims

| Place of search | Date of completion of the search | Examiner |
|---|---|---|
| Munich | 8 June 2021 | Cismaru, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | "UTILITY OF ALCOHOLS AND OTHER OH CONTAINING INGREDIENTS TO MODIFY VISCOSITY OF SUNSCREEN GELS CONTAINING SPECIALTY POLYMERS ED - Darl Kuhn", IP.COM, IP.COM INC., WEST HENRIETTA, NY, US, 24 August 2011 (2011-08-24), XP013147129, ISSN: 1533-0001 * page 10; examples * | 1-15 | |
| Y | WO 2018/194086 A1 (KAO CORP [JP]) 25 October 2018 (2018-10-25) * claims * * examples * | 1-15 | |

|  |  |
|---|---|
|  | TECHNICAL FIELDS SEARCHED (IPC) |

The present search report has been drawn up for all claims

| Place of search | Date of completion of the search | Examiner |
|---|---|---|
| Munich | 8 June 2021 | Cismaru, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 21 4423

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2021

| Patent document cited in search report | Publication date | Patent family member(s) | | Publication date |
|---|---|---|---|---|
| DE 102013215831 A1 | 12-02-2015 | DE | 102013215831 A1 | 12-02-2015 |
| | | EP | 3030214 A1 | 15-06-2016 |
| | | JP | 2016527288 A | 08-09-2016 |
| | | WO | 2015018549 A1 | 12-02-2015 |
| US 2014212363 A1 | 31-07-2014 | AU | 2012304368 A1 | 06-02-2014 |
| | | BR | 112014005288 A2 | 28-03-2017 |
| | | CN | 103781461 A | 07-05-2014 |
| | | EP | 2753293 A1 | 16-07-2014 |
| | | JP | 6117791 B2 | 19-04-2017 |
| | | JP | 2014526470 A | 06-10-2014 |
| | | KR | 20140072080 A | 12-06-2014 |
| | | TW | 201325630 A | 01-07-2013 |
| | | US | 2014212363 A1 | 31-07-2014 |
| | | WO | 2013036878 A1 | 14-03-2013 |
| WO 2018194086 A1 | 25-10-2018 | CN | 110520100 A | 29-11-2019 |
| | | JP | 2018177796 A | 15-11-2018 |
| | | KR | 20190137096 A | 10-12-2019 |
| | | TW | 201841607 A | 01-12-2018 |
| | | WO | 2018194086 A1 | 25-10-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9411119 A
- WO 2001012139 A
- US 20100116897 A
- DE 102017108610
- EP 19197645 A
- DE 102017108612
- DE 102017108613
- DE 102107108614
- DE 102107108615
- US 20140212363 A
- WO 0112139 A

### Non-patent literature cited in the description

- **J. SANGSTER.** Octanol-Water Partition Coefficients: Fundamentals and Physical Chemistry. Wiley Series in solution chemistry, John Wiley & Sons, 1997, vol. 2
- *CHEMICAL ABSTRACTS,* 288254-16-0